# EUROPEAN PATENT SPECIFICATION

(11) **EP 0 306 210 B1**
(45) Date of publication and mention of the grant of the patent: **29.09.1993**
(21) Application number: 88307858.6
(22) Date of filing: 24.08.1988
(51) Int. Cl.: C07D 309/30, C07F 7/18, B01J 31/24, A61K 31/35

(54) **A novel hydrogenation process for the formation of di or tetrahydro HMG-CoA reductase inhibitors**
Hydrierverfahren zur Bildung von Di- oder Tetrahydro-HMG-CoA-Reduktase-Inhibitoren
Procédé d'hydrogénation pour la formation d'inhibiteurs de la réductase de la di- ou tétrahydro HMG-CoA

(30) Priority: 03.09.1987 US 92804; 03.09.1987 US 92802; 03.09.1987 US 92803; 03.09.1987 US 92801
(43) Date of publication of application: 08.03.1989
(73) Proprietor: Merck & Co., Inc., Rahway New Jersey 07065-0900 (US)
(72) Inventor: DeCamp, Ann Elizabeth,, New Providence New Jersey 07974 (US); Verhoeven, Thomas R., Cranford New Jersey 07016 (US); Shinkai, Ichiro, Westfield New Jersey 07090 (US)
(74) Representative: Cole, William Gwyn

(56) References cited:
- EP-A- 0 033 537
- EP-A- 0 137 444
- DD-A- 219 956
- CHEMICAL ABSTRACTS, vol. 98, no. 11, March 14, 1983, Columbus, Ohio, USA, SANKYO CO. "Dihydro ML-236B, dihydro MB-530B, and their derivates", page 540, column 1, Abstract-no. 89 173c

## Description

### BACKGROUND OF THE INVENTION

Lovastatin and derivatives therefrom (e.g. Simvastatin) are potent HMG-CoA reductase inhibitors. Lovastatin is produced in fermentations with Aspergillus terreus as described in U.S. Patent 4,231,938 by Monaghan et al. A 4a,5-dihydrolovastatin (i) is co-produced with lovastatin in considerably 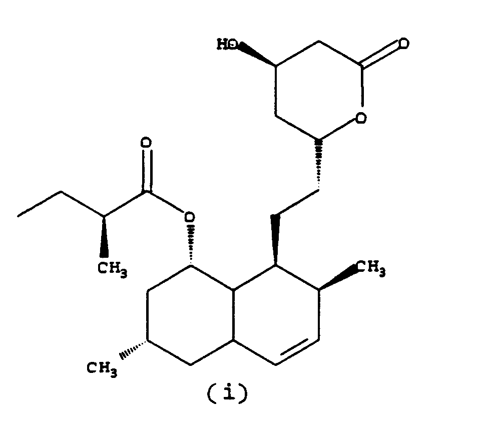
lower yield as described in U.S. Patent No. 4,294,846 by Albers-Schonberg et al. However, this naturally occurring 4a,5-dihydromevinolin appears to be slightly more active than lovastatin as an HMG-CoA reductase inhibitor. Efforts to convert the more abundant lovastatin to its more active 4a,5-dihydro derivative have been made by catalytic reduction as reported in published EP application No. 0033537 but no evidence of the desired transfused 4a,5-dihydrolovastatin was found. Kuo et al. (U.S. Patent 4,490,546 and J. Org. Chem. 48, 1991 (1983)) have disclosed a process for hydrogenating the 4a,5 double bond of lovastatin. However this process requires 5 separate steps leading to the natural trans isomer in about 10 percent yield. Moreover the desired product is contaminated with the cis-fused dihydro derivative.

Patchett et al. (U.S. Patent 4,351,844) have found that a 3,4-dihydrolovastatin analog (ii) a 3,5-dihydrolovastatin (iii) and a trans-fused tetrahydro derivative (iv) are potent HMG-CoA reductase inhibitors. 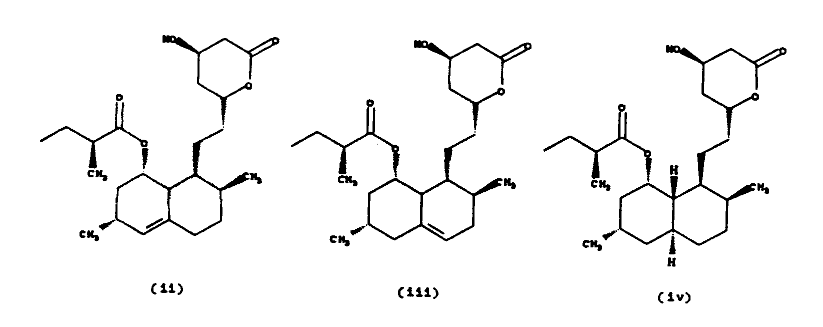
Patchett, supra, describe a catalytic hydrogenation process employing tris(triphenylphosphine) chlororhodium in an aromatic solvent for the reduction of lovastatin to compound (ii). This process has also been described by Kuo et al., J. Org. Chem., 48, 1991 (1983). The Patchett-Kuo process in an aromatic solvent requires a large excess (225 to 100 weight percent catalyst to lovastatin) of the expensive rhodium catalyst. This method also produces approximately 10 percent of the isomeric 3,5-dihydrolovastatin derivative (iii) which required several recrystallizations and preparative HPLC for separation. When the Patchett-Kuo process is applied to synvinolin (simvastatin) 20 percent of the isomeric 3,5 dihydro simvastatin derivative is produced.

Patchett, et al., supra, describe a catalytic hydrogenation process, employing palladium on calcium carbonate in absolute ethanol, for the reduction of lovastatin to a compound of structure (iii). This process has also been described by Kuo et al. J. Org. Chem, 48, 1991(1983). This palladium catalyzed hydrogenation has been described as yielding the 3,5-dihydrolovastatin together with varying amounts of the 3,4-dihydro isomer (ii) as a contaminant. Kuo et al, supra, also describe an alternative procedure for the synthesis of compound (iii) vis-vis the treatment of tertbutyldimethylsilyl-lovastatin with triethyl silane in methylene chloride followed by protolysis with trifluoroacetic acid. However, this process requires silylation-desilylation and results in low yields of the desired 3,5-dihydro reduction product.

Patchett et al. disclose a catalytic hydrogenation procedure for preparing (iv) from lovastatin. This process involves hydrogenation over platinum oxide in ethyl acetate. However, the product formed, at best, is a mixture of 60% trans and 40% cis-tetrahydrolovastatin. The desired trans isomer could be isolated from this mixture only after silica gel chromatographic purification.

Sletzinger et al. (U.S. Patent 4,584,389) improved on the trans/cis ratio in the reduction product mixture by using platinum on alumina as the hydrogenation catalyst. Using this catalyst the yield of trans-fused product was increased to 65-75%, however, this process also produced a partially reduced dihydro by-product which although present in small amounts (1-5%) was quite difficult to remove by either chromatography or crystallization.

### DETAILED DESCRIPTION OF THE INVENTION

This invention may be depicted as a process for the preparation of a compound of structural formula (I): 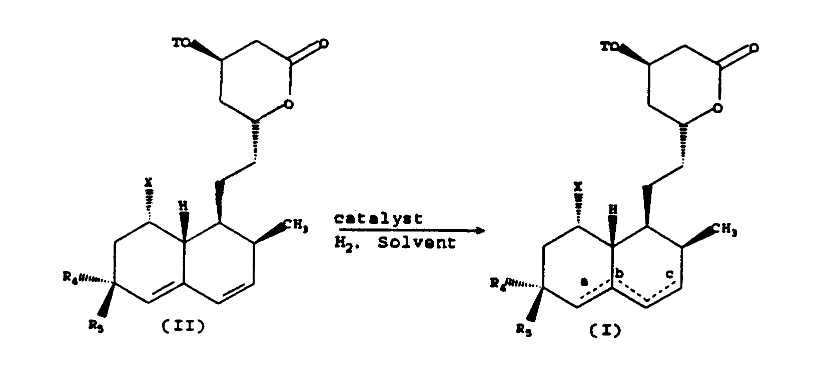
wherein:
- T is: H or t-C₄H₉(CH₃)₂Si;
- X is: 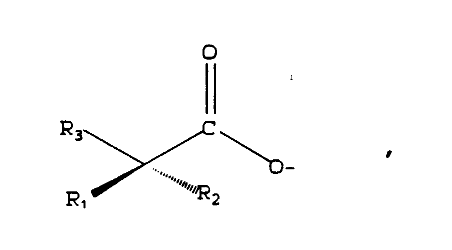 OH, NH₂, or OR₆;
- R₁ is: H or C₁₋₃alkyl;
- R₂ is: H or C₁₋₃alkyl;
- R₃ is: C₁₋₅alkyl, phenyl, or C₃₋₇ cycloalkyl; or C₁₋₅alkyl or phenyl substituted with a group Y where Y is selected from:
a) OH or t-C₄H₉(Me)₂SiO;
b) halogen;
c) trifluoromethyl;
d) C₁₋₃alkoxy;
e) C₁₋₃alkylcarbonyloxy;
f) phenylcarbonyloxy;
g) C₁₋₃alkoxycarbonyl; or
h) phenyloxycarbonyl;
- R₄ is: H, CH₃, CH₂OH, OH, CH₂OSi(Me)₂t-C₄H₉ or OSi(Me)₂t-C₄H₉;
- R₅ is: H, CH₂OH, OH, CH₂OSi(Me)₂t-C₄H₉ or OSi(Me)₂t-C₄H₉; provided that when either R₄ or R₅ is CH₂OH or CH₂OSi(Me)₂t-C₄H₉ the other is H; and one and only one of R₄ and R₅ can be OH or OSi(Me)₂t-C₄H₉;
- R₆ is: C₁₋₅alkyl, C₃₋₇cycloalkyl, phenylC₁₋₃alkyl, or substituted phenylC₁₋₃alkyl in which the substituent is halogen, CN or CF₃;
- a, b, c: are double bonds or single bonds provided that only one of a, b or c is a double bond;
which comprises:
contacting of a compound of structural formula (II): 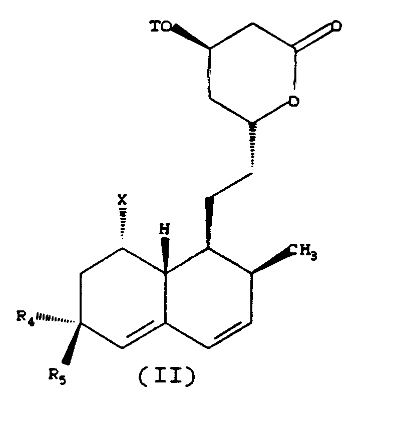
with a homogenous metal catalyst, in an organic solvent under a pressure of hydrogen gas at a temperature of 25-80°C provided that where a is a double bond:
- X is: 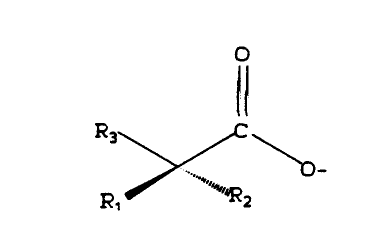
- T is: H; and
the homogenous metal catalyst is Rh(Ar₃P)₃ Z,
wherein
- Ar is: phenyl or naphthyl or C₁₋₃alkoxyphenyl or C₁₋₃alkoxynapthyl;
- Z is: Cl or Br;
the organic solvent is
an alcoholic solvent; and the pressure is 2.75x10⁵-9.65x10⁶ Nm⁻² (40-1400 psi) at a temperature of 40-80°C; and
where b is a double bond:
- X is: 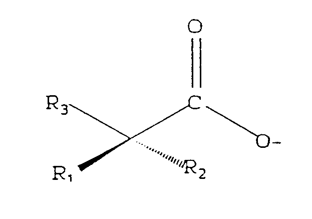
- T is: H; and
the homogenous metal catalyst is 1,5-cyclooctadiene(pyridine)(tricyclohexyl phosphine)iridium(I)hexafluorophosphate or norbornadiene-1,4-bis-(diphenylphosphino)butane-rhodium(I)-tetrafluoroborate;
the organic solvent is selected from dichloromethane, chloroform or chlorobenzene; and the pressure is one atmosphere hydrogen at a temperature of 25 to 80°C; and
where c is a double bond:
- X is: OH, NH₂ or OR₆;
- T is: t-C₄H₉(CH₃)₂Si,
the homogenous metal catalyst is 1,5-cyclooctadiene(pyridine)(tricyclohexyl phosphine)iridium(I)hexafluorophosphate or norbornadiene-1,4-bis-(diphenylphosphino)butane-rhodium(I)-tetrafluoroborate;
the organic solvent is an alcoholic solvent; and the pressure is 1.013x10⁵ Nm⁻² (one atmosphere) hydrogen at ambient temperature; and
where a, b and c are all single bonds:
- X is: OH, NH₂, or OR₆;
- T is: t-C₄H₉(CH₃)₂Si,
the homogenous metal catalyst is 1,5-cyclooctadiene(pyridine)(tricyclohexyl phosphine)iridium(I)hexafluorophosphate or norbornadiene-1,4-bis-(diphenylphosphino)butane-rhodium(I)-tetrafluoroborate;
the organic solvent is selected from dichloromethane or chloroform, or chlorobenzene; the pressure is 1.03x10⁵-2.76x10⁵ Nm⁻² (15-40 psi) at a temperature of about 25-80°C.

The instant process provides for the reduction of both double bonds in II or a selective reduction so that a monoene (I) with a, b or c as a double bond is formed.

To selectively reduce the 3,4 double bond in the polyhydronaphthyl ring of lovastatin, simvastatin or 8-acyl or 6-substituted analogs thereof a compound (II) wherein X is an acyl moiety is employed with the Rh(Ar₃P)₃Z catalyst and an alcoholic solvent. A homogenous rhodium catalyst is able to discriminate between a double bond in the 3,4 and 4a,5 positions, complexing with and thus allowing hydrogenation of only the 3,4 double bond. Critical to the present invention is applicants' finding that an alcoholic solvent such as isopropanol or ethanol or a mixture of such alcohol with a hydrocarbon such as benzene or tolune, increases the catalyst lifetime which thus removes the necessity of large excesses of the expensive rhodium catalyst. The present reaction is typically conducted with a 5 weight percent of catalyst to olefinic substrate which contrasts with the very large excesses of catalyst required in the prior art reductions. Unexpectedly, inclusion of an alcoholic co-solvent also decreases substantially the amount of the "1,4-reduction" side product (e.g. iii) which is produced. Formation of large proportions of this 3,5-dihydro side product is highly detrimental and seriously compromises the final product purity since it can co-crystallize with the desired product. This side product has previously been removed by a tedious HPLC procedure.

The instant process can be employed to selectively reduce the 4a,5 double bond in the polyhydronaphthyl ring of des-(α-methylbutyryl)-8-hydroxy lovastatin or analogs thereof. In this selective reduction, the C-8 hydroxyl, or a comparable ligating group such as OR or NH₂ is utilized to direct the delivery of hydrogen to the more sterically hindered 4a,5 double bond. The agent used to effect this directed hydrogen transfer is a homogenous iridium or rhodium catalyst. Critical to the reduction of the 4a,5 double bond is the utilization of an alcoholic cosolvent, such as a mixture of dichloromethane, chloroform or chlorobenzene or a like substance and an alcohol such as isopropanol or ethanol, to modify catalyst activity and thus minimize formation of a tetrahydro derivative. The inclusion of an alcoholic cosolvent in the reaction mixture increases the ratio of product to tetrahydro derivative from 3:2 to 9:1. The instant process yields a single dihydro product. No dihydro products derived from reduction of the 3,4 double bond are observed; furthermore, double bond isomerization which could lead to formation of the 4,4a double bond is not observed.

The instant process can also be employed to selectively add hydrogen to the 3,5 positions in the polyhydronaphthyl ring of lovastatin, simvastatin or C-8-acyl or C-6-substituted analogs thereof. To obtain this selectivity, X on the starting material (II) should be an acyl moiety, the catalyst a homogenous iridium or rhodium catalyst and the solvent dichloromethane, chloroform, chlorobenzene or a like substance. A homogenous iridium or rhodium catalyst as described herein, is, in the absence of a hydroxyl-coordination site at the C-8-position, able to distinguish between a 1,2 and a 1,4 hydrogenation, allowing only the 1,4 reduction under the herein described conditions. The 3,5-dihydro-product is formed in high yield uncontaminated by any other dihydro isomer.

The instant process can also be used to reduce both double bonds of des-(α-methylbutyryl)-C-8-hydroxy-lovastatin and C-8-amino, C-8-alkoxy and C-6-substituted analogs. The present invention provides for formation of the trans-fused tetrahydro product (e.g. IV) in essentially 100% yield and uncontaminated (<1%) with any dihydro derivatives.

The invention may be practiced wherein R₄ or R₅ are unprotected CH₂OH or OH moieties, however in some instances it may be preferable to protect OH with a hydroxyl protecting group such as tert-butyldimethylsilyl. Other protecting groups such as tert-butyldiphenylsilyl, trimethylsilyl, triethylsilyl, triisopropylsilyl and tetrahydropyranyl could be substituted for tert-butyldimethylsilyl without effecting the outcome of the instant invention.

Where necessary a tert-butyldimethylsilyl protecting group is also employed to mask the 4-hydroxy group in the lactone moiety. As indicated above, other hydroxyl protecting groups may be used with equal effectiveness.

One embodiment of the present invention is a process for the preparation of a compound of structural formula (Ia): 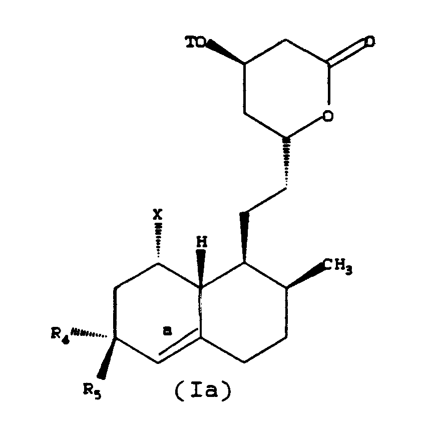
wherein:
- T is: H;
- X is: 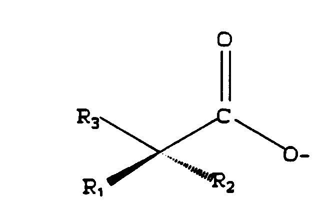
- R₁ is: methyl,
- R₂ is: H or C₁₋₃alkyl,
- R₃ is: C₁₋₅alkyl, phenyl or C₃₋₇cycloalkyl; or C₁₋₅alkyl or phenyl substituted with a group Y where Y is a group not reduced by a triarylphosphine rhodium halide such as:
a) OH or t-C₄H₉(Me)₂SiO;
b) halogen (F, Cl or Br);
c) trifluoromethyl;
d) C₁₋₃alkoxy;
e) C₁₋₃alkylcarbonyloxy;
f) phenylcarbonyloxy;
g) C₁₋₃alkoxycarbonyl; or
h) phenyloxycarbonyl;
- R₄ is: H, CH₃, CH₂OH, OH, CH₂OSi(Me)₂t-C₄H₉ or OSi(Me)₂t-C₄H₉;
- R₅ is: H, CH₂OH, OH, CH₂OSi(Me)₂t-C₄H₉ or OSi(Me)₂t-C₄H₉; provided that when either of R₄ and R₅ is CH₂OH or CH₂OSi(Me)₂t-C₄H₉ the other is H;
which comprises:
contacting of a compound of structural formula (II): 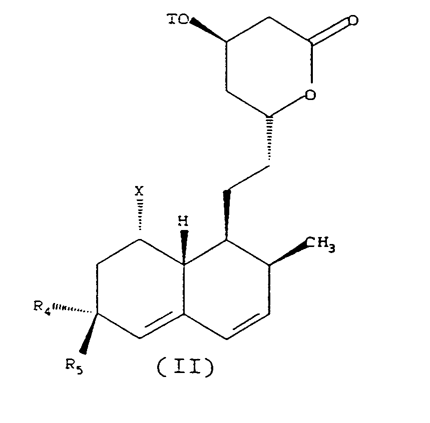
with Rh(Ar₃P)₃Z; wherein
- Ar is: phenyl or naphthyl or C₁₋₃alkoxy substituted phenyl or naphthyl;
- Z is: Cl or Br;
in an alcoholic solvent, under hydrogen gas at a pressure of 2.76x10⁵-9.65x10⁶ Nm⁻² (40-1400 psi) at a temperature of 40-80°C.

In one class of this embodiment the process is used to form compounds (Ia) wherein:
- R₂ is: H or CH₃;
- R₃ is: CH₃CH₂;
- R₄ is: H, CH₃, CH₂OH or CH₂OSi(Me)₂t-C₄H₉;
- R₅ is: H, CH₂OH, or CH₂OSi(Me)₂t-C₄H₉;
provided that at least one of R₄ or R₅ is H. Exemplifying this class are those compounds (Ia) selected from the group wherein:
a. R₂ is CH₃, R₄ is CH₃, R₅ is H;
b. R₂ is H, R₄ is CH₃, R₅ is H;
c. R₂ is CH₃, R₄ is CH₂OH, R₅ is H;
d. R₂ is CH₃, R₄ is H, R₅ is CH₂OH;
e. R₂ is H, R₄ is CH₂OH, R₅ is H;
f. R₂ is H, R₄ is H, R₅ is CH₂OH;
g. R₂ is CH₃, R₄ is CH₂OSi(Me)₂t-C₄H₉, R₅ is H;
h. R₂ is CH₃, R₄ is H, R₅ is CH₂OSi(Me)₂t-C₄H₉.
The rhodium catalyst of this embodiment is a tris(triaryl or substituted aryl phosphine) rhodium halide, preferably tris(triphenylphosphine) rhodium bromide or chloride, most preferably the chloride. The solvent is isopropanol or ethanol or a mixture of said alcohol with toluene or benzene or a like hydrocarbon. An example of such a mixture is 50-50 percent by volume isopropanol:toluene. The preferable solvent is isopropanol. The diene substrate and the rhodium catalyst, in a weight percent ratio of about 5-75 weight percent catalyst to substrate, preferably about 5-25 percent, most preferably about 5 percent catalyst to substrate, are dissolved in the alcoholic solvent, at a temperature of 25° to 80°C, preferably 40°C under hydrogen gas at a pressure of 2.76x10⁵-9.65x10⁶ Nm⁻² (40-1400 psi), preferably 4.14x10⁶-9.65x10⁶ Nm⁻² (600-1400 psi) for about 25 hours. After standard workup procedures, evaporation of solvent gives a crystalline solid which can be recrystallized from ethyl acetate/hexanes to yield product containing less than 1 percent of olefin from a 1,4 reduction.

A second embodiment of the instant invention is a process for the preparation of a compound of structural formula (Ic): 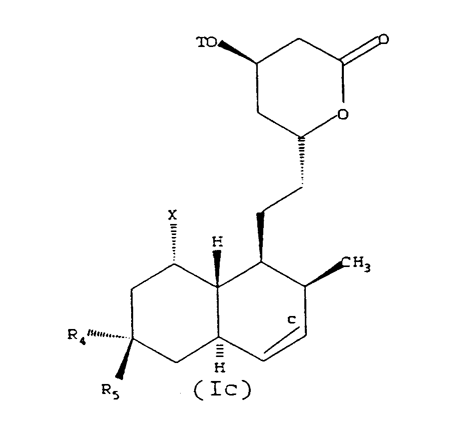
wherein:
- T is: t-C₄H₉(Me)₂Si;
- X is: OH, NH₂, or OR₆; wherein R₆ is C₁₋₅ alkyl, C₃₋₇ cycloalkyl, phenyl C₁₋₃ alkyl, or substituted phenyl C₁₋₃ alkyl in which the substituent is halogen, CF₃ or CN;
- R₄ is: H, CH₃, CH₂OSi(Me)₂t-C₄H₉ or Osi(Me)₂t-C₄H₉;
- R₅ is: H, CH₂OSi(Me)₂t-C₄H₉ or OSi(Me)₂t-C₄H₉; provided that when R₄ or R₅ is CH₂OSi(Me)₂t-C₄H₉ the other is H; and one and only one of R₄ and R₅ can be OSi(Me)₂t-C₄H₉;
which comprises:
contacting a compound of structural formula (II): 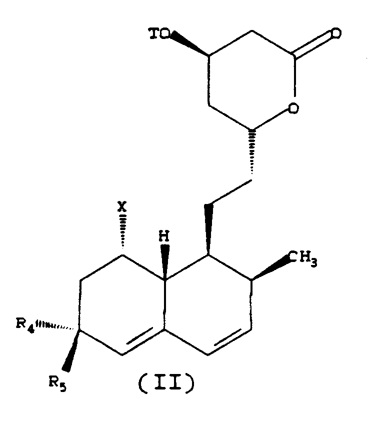
with 1,5-cyclooctadiene(pyridine)(tricyclohexylphosphine)iridium (I) hexafluorophosphate or norbornadiene-1,4-bis(diphenylphosphino)butane rhodium (I) hexafluoroborate; in an alcoholic solvent, under hydrogen gas pressure.

In one class of this embodiment the process is used to form compounds (Ic) wherein:
- X is: OH,
Exemplifying this class are those compounds (Ic) selected from the group wherein:
a. R₄ is CH₃, R₅ is H;
b. R₄ is CH₂OSi(Me)₂t-C₄H₉, R₅ is H;
c. R₄ is H, R₅ is CH₂OSi(Me)₂t-C₄H₉;
d. R₄ is OSi(Me)₂t-C₄H₉, R₅ is H;
e. R₄ is H, R₅ is OSi(Me)₂t-C₄H₉;
f. R₄ is CH₃, R₅ is OSi(Me)₂t-C₄H₉;
The iridium catalyst Ir[(COD)PCy₃(pyr)]PF₆. (COD=1,5-cyclooctadiene, PCy₃=tricyclohexylphosphine, pyr=pyridine) can be prepared following the procedure of Crabtree et al., J. Organomet. Chem., 135, 395 (1977). The rhodium catalyst [Rh(NBD)(DIPHOS-4)]BF₄ (NBD=norbornadiene, DIPHOS-4=1,4-bis(diphenylphosphino)butane) can be prepared following the procedure described by Stille et al. J. Org. Chem. 47., 468 (1982) and further detailed by Evans et al. as reported in J. Am. Chem. Soc., 106, 3866 (1984). The hydrogenation procedure for this embodiment may use the above described iridium or rhodium catalyst or a like catalyst capable of complexing with the C-8 hydroxyl, ether or amino functionality; the preferred catalyst is the iridium catalyst. The relative mole percent of catalyst to olefinic substrate may be 0.1 to 10 mole percent, preferably about 2.5 mole percent. The alcoholic solvent for this embodiment is a mixture of dichloromethane, chloroform or chlorobenzene or a like substance and a lower alkyl chain alcohol such as ethanol or isopropanol. The preferred mixture is dichloromethane and isopropanol. The relative proportions of halogenated hydrocarbon to alcohol may be about 20:1 to about 2:1 by volume, preferably about 6:1. The relative ratio of equivalents of alcohol to equivalent olefin is 0.5:1 to 10:1, preferably 3:1.

The olefinic substrate and catalyst are dissolved in the solvent and the mixture reduced under atmospheric hydrogen pressure at ambient temperature for about 20 hours. The reaction mixture is worked up following standard procedures to yield a 9:1 mixture of the 4a,5-dihydro to trans tetrahydro derivative. Further purification is accomplished by chromatography of this mixture; where X is OH, the hydroxyl group may first be acylated and then flash chromatographed with a 25% by volume ethyl acetate:hexane mixture.

A third embodiment of the instant invention is a process for the preparation of a compound of structural formula (Ib): 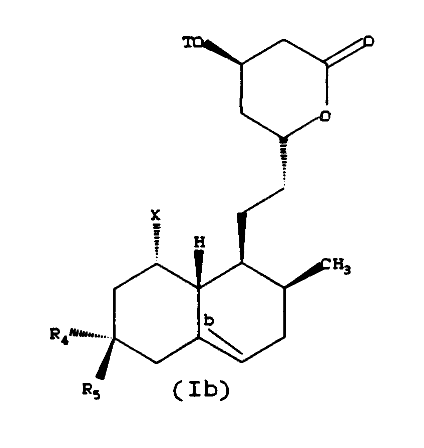
wherein:
- T is: H;
- X is: 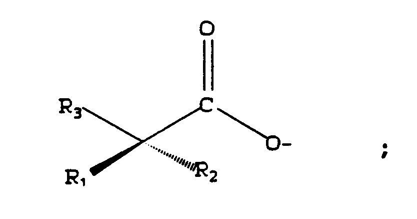
- R₁ is: CH₃;
- R₂ is: H or C₁₋₃alkyl;
- R₃ is: C₁₋₅alkyl, phenyl, or C₃₋₇ cycloalkyl; or C₁₋₅alkyl or phenyl substituted with a group Y where Y is a group not reduced by the catalyst herein, examples of such a group Y are:
a) t-C₄H₉(Me)₂SiO-;
b) halogen (F, Cl or Br);
c) trifluoromethyl;
d) C₁₋₃ alkoxy;
e) C₁₋₃alkylcarbonyloxy;
f) phenylcarbonyloxy;
g) C₁₋₃alkoxycarbonyl; or
h) phenyloxycarbonyl;
- R₄ is: H, CH₃, CH₂OSi(Me)₂t-C₄H₉ or OSi(Me)₂t-C₄H₉;
- R₅ is: H, CH₂OSi(Me)₂t-C₄H₉ or
OSi(Me)₂t-C₄H₉; provided that
when either R₄ or R₅ is CH₂OSi(Me)₂t-C₄H₉ the other is H; and one and only one of R₄ and R₅ can be OSi(Me)₂t-C₄H₉;
which comprises:
contacting a compound of structural formula (II): 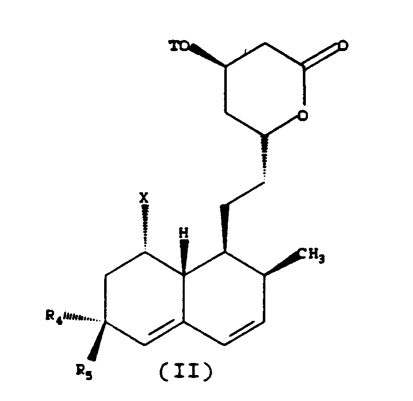
with 1,5-cyclooctadiene(pyridine)(tricyclohexylphosphine)iridium (I) hexafluorophosphate or norbornadiene-1,4-bis(diphenylphosphino)butane rhodium (I) hexafluoroborate; in a solvent, under an atmospheric pressure of hydrogen gas at 25° to 80°C.

In one class of this embodiment the process is used to form compounds (Ib) wherein:
- R₂ is: H or CH₃;
- R₃ is: CH₃CH₂;
- R₄ is: H, CH₃, or CH₂OSi(Me)₂t-C₄H₉;
- R₅ is: H, or CH₂OSi(Me)₂t-C₄H₉;
provided that at least one of R₄ or R₅ is H. Exemplifying this class are those compounds (Ib) selected from the group wherein:
a. R₂ is CH₃, R₄ is CH₃, R₅ is H;
b. R₂ is H, R₄ is CH₃, R₅ is H;
c. R₂ is CH₃, R₄ is CH₂OSi(Me)₂t-C₄H₉, R₅ is H;
d. R₂ is CH₃, R₄ is H, R₅ is CH₂OSi(Me)₂t-C₄H₉;
e. R₂ is H, R₄ is CH₂OSi(Me)₂t-C₄H₉, R₅ is H;
f. R₂ is H, R₄ is H, R₅ is CH₂OSi(Me)₂t-C₄H₉.

The catalyst of this embodiment is [Ir(COD)PCy₃(pyr)]PF₆ (COD=1,5-cyclooctadiene, PCy₃=tricyclohexylphosphine, pyr=pyridine) or [Rh(NBD)(DIPHOS-4)]BF₄. (NBD=norbornadiene, DIPHOS-4=1,4 bis(diphenylphosphino)butane), preferably [Ir(COD)PCy₃(pyr)]PF₆. The diene substrate and the iridium or rhodium catalyst in a mole percent of 0.1 to 10 mole percent catalyst to diene, preferably 2.5 mole percent catalyst to diene, is dissolved in dichloromethane, chloroform, chlorobenzene or a like solvent, preferably dichloromethane. The solution is reduced under atmospheric hydrogen pressure at a temperature of 25° to 80°C, preferably 25°C. After standard workup procedures, evaporation of solvent yields the desired 3,5-dihydro derivative.

A fourth embodiment of the instant invention is a process for the preparation of a compound of structural formula (Id): 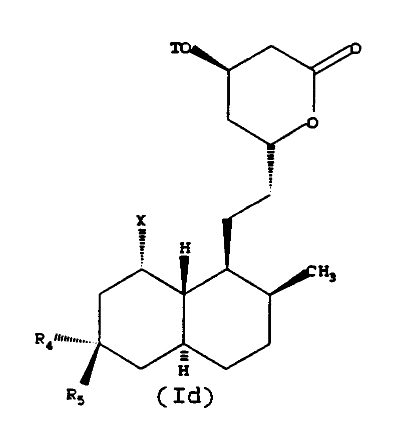
wherein:
- T is: t-C₄H₉(CH₃)₂Si;
- X is: OH, NH₂ or OR; wherein R is C₁₋₅ alkyl, C₃₋₇ cycloalkyl, phenyl-C₁₋₃ alkyl, or substituted phenyl-C₁₋₃ alkyl in which the substituent is halogen, CF₃ or CN;
- R₄ is: H, CH₃, CH₂OSi(Me)₂t-C₄H₉ or OSi(Me)₂t-C₄H₉;
- R₅ is: H or CH₂OSi(Me)₂t-C₄H₉ or OSi(Me)₂t-C₄H₉;
provided that when R₄ or R₅ is CH₂OSi(Me)₂t-C₄H₉ the other is H;
and one and only one of R₄ and R₅ can be OSi(Me)₂t-C₄H₉;
which comprises:
contacting a compound of structural formula (II): 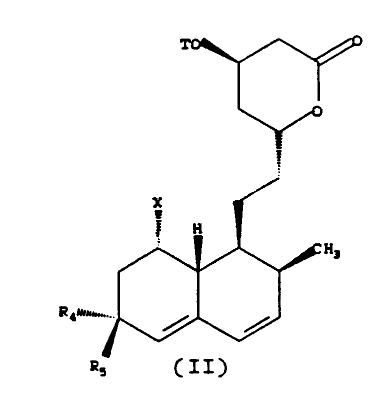
with 1,5-cyclooctadiene(tricyclohexylphosphine) (pyridine)iridium(I) hexafluorophosphate or norbornadiene-1,4-bis(diphenylphosphino)butane rhodium (I) tetrafluoroborate in an organic solvent at a temperature of about 25-80°C under a hydrogen gas pressure of about 1.03x10⁵-2.76x10⁵ Nm⁻² (15-40 psi).

In one class of this embodiment the process is used to form compounds (Id) wherein:
- X is: OH;
- R₄ is: H, CH₃, CH₂OSi(Me)₂t-C₄H₉ or OSi(Me)₂t-C₄H₉;
- R₅ is: H, CH₂OSi(Me)₂t-C₄H₉ or OSi(Me)₂t-C₄H₉;
provided that when one of R₄ and R₅ is CH₂OSi(Me)₂t-C₄H₉ the other is H. Exemplifying this class are those compounds (Id) selected from the group wherein:
a. R₄ is CH₃, R₅ is H;
b. R₄ is CH₂OSi(Me)₂t-C₄H₉, R₅ is H;
c. R₄ is H, R₅ is CH₂OSi(Me)₂t-C₄H₉;
d. R₄ is OSi(Me)₂t-C₄H₉, R₅ is H;
e. R₄ is H, R₅ is OSi(Me)₂t-C₄H₉;
f. R₄ is CH₃, R₅ is OSi(Me)₂t-C₄H₉.

The catalyst of this embodiment may be [Ir(COD)PCy₃(pyr)]PF₆ (COD = 1,5-cyclooctadiene, PCy₃ = tricyclohexylphosphine, pyr = pyridine) or [Rh(NBD)(DIPHOS-4)]BF₄ (NBD = Norbornadiene, DIPHOS-4 = 1,4-bis(diphenylphosphino)butane), preferably [Ir(COD)PCy₃(pyr)]PF₆. The organic solvent is dichloromethane or chloroform or chlorobenzene or a like substance, preferably dichloromethane. The diene (II) and catalyst in approximately 0.5-10.0 mole percent catalyst to substrate, preferably 2.5 mole percent are dissolved in an organic solvent at a temperature of 25°-80°C, preferably 25°C under a pressure of 1.03x10⁵-2.76x10⁵ Nm⁻² (15-40 psi), preferably 2.76x10⁵ Nm⁻² (40 psi) for about 24 hours. The solution is worked up following standard procedures and the solvent evaporated to yield a crystalline product.

Starting diene, lovastatin, wherein R₁ is methyl, R₂ is hydrogen, R₃ is ethyl, R₄ is methyl, R₅ is hydrogen is readily available or may be prepared according to the fermentation procedures disclosed in U.S. Patent 4,231,938. Simvastatin, wherein R₁ is methyl, R₂ is methyl, R₃ is ethyl, R₄ is methyl, R₅ is hydrogen may be prepared from lovastatin following the procedure described in U.S. Patent 4,582,915 or copending U.S. application Serial No. 072066 filed July 10, 1987.

Starting dienes wherein X is an acyl moiety and wherein R₄ or R₅ is CH₂OH are prepared following the procedure outlined in copending U.S. Patent application S.N. 048136 filed May 15, 1987. Compounds wherein R₄ or R₅ is OH can be prepared following the descriptions in U.S. Patents 4,517,373 and 4,537,859 for preparing the 6-hydroxyl derivatives. Protection of the 6-hydroxymethyl or the 6-hydroxy group can be accomplished following the procedure outlined in U.S. Patent 4,444,784.

Dienes where X is an acyl moiety and where both R₄ and R₅ are hydrogen may be prepared from the fermentation product compactin (also known as mevastatin) (Endo, et. al. J. Antibiot, 29, 1346 (1976)).

Starting dienes with substituted acyl groups are prepared using acyl chlorides, prepared by standard techniques, and the acylation procedure described by Hoffman et. al., in U.S. Patent 4,444,784 or that disclosed in copending U.S. Patent application S.N. 038560 filed April 15, 1987.

Starting diene wherein X is OH and R₄ is CH₃ is readily prepared from lovastatin following the hydrolysis conditions disclosed in U.S. Patent 4,444,784. The 4-hydroxy function in the lactone moiety is protected with a suitable protecting agent, exemplified here as a t-butyldimethylsilyl group, according to the procedure disclosed in U.S. Patent 4,444,784. Starting dienes wherein X is OH and R₄ or R₅ is CH₂OSi(Me)₂t-C₄H₉ are prepared following the procedure outlined in copending U.S. Patent application S.N. 048136 filed May 15, 1987, followed by protection with t-C₄H₉(Me)₂SiCl.

Starting dienes wherein X is OR₆ can be prepared following the procedure described in U.S. Patent 4,282,155; where X is NH₂ the olefins can be prepared following the procedure in U.S. Patent 4,472,426.

Starting olefins where R₄ or R₅ is OSi(Me)₂t-C₄H₉ can be prepared following the descriptions in U.S. Patents 4,517,373 and 4,537,859.

The reduced products (I) wherein X is OH can be readily modified through esterification of the C-8 hydroxyl group and removal of protecting groups to yield a variety of 8-acyloxy derivatives. This esterification can be accomplished following the acylation procedure disclosed by Hoffman et al. U.S. Patent 4,444,784 or that described in copending U.S. application S.N. 038580 filed April 15, 1987.

Products (I) wherein X is NH₂ can be converted into 8-amido derivatives following the procedure exemplified in U.S. Patent 4,472,426. The 8-amido derivatives are disclosed in the above patent as HMG-CoA reductase inhibitors.

Products (I) wherein X is OR₆ are disclosed in U.S. Patent 4,282,155 as HMG-CoA reductase inhibitors.

Tert-butyldimethylsilyl is shown as a hydroxyl protecting group. It will be clear to those skilled in the art that other hydroxyl protecting groups such as tert-butyldiphenylsilyl, trimethylsilyl, triethylsilyl, triisopropylsilyl and tetrahydropyranyl could be substituted for tert-butyldimethylsilyl without effecting the outcome of the instant invention.

The following Examples illustrate the present invention and as such are not to be considered as limiting the invention set forth in the claims appended hereto.

### EXAMPLE 1

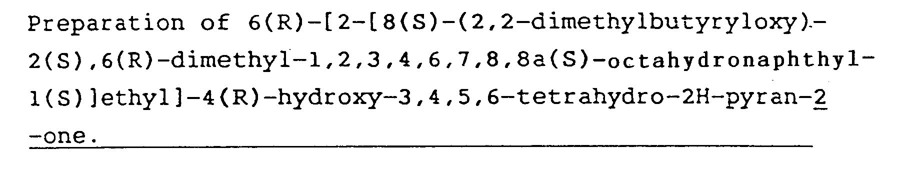

A solution of 6(R)-[2-[8(S)-(2,2-dimethylbutyryloxy)-2(S),6(R)-dimethyl-1,2,6,7,8,8a(R)-hexahydronaphthyl-1(S)]ethyl]-4(R)-hydroxy-3,4,5,6-tetrahydro-2H-pyran-2-one (0.302 g, 0.722 mmol) and tris(triphenyl phosphine)rhodium (I) chloride (0.015 g 0.016 mmol) in isopropanol (25 ml) was reduced at 40°C under 9.65x10⁵-4.14x10⁶ Na (1400 to 600 psi) of H₂ for 25 hours. The solution was evaporated in vacuo and the residue taken up in diethyl ether and stirred. A precipitate formed. The slurry was vacuum filtered through a 1 inch bed of Florisil® (Magnesium silicate filter aid used herein to retain any catalyst complex which remained in solution) and washed with 35 ml diethyl ether. The filtrate was evaporated in vacuo to a foamy glass. Further purification was achieved by short path silica gel chromatography (5-10 g silica gel/gram crude product) eluting with 1 percent isopropanol in dichloromethane. Evaporation of the appropriate fractions gave a white crystalline solid which was recrystallized from ethyl acetate/hexanes to give the title compound as white needles (m.p. 123-123.5°C). The product contained less than one percent of the "1,4 reduction" side product. HNMR (300 MHz, CDCl₃) δ 5.38 (m, 1H), 5.32 (m, 1H); 4.59 (m, 1H); 4.34 (m, 1H); 2.72 (dd, 1H, J=17.7, 5.4 Hz); 2.62 (m, 2H), 2.17-2.37 (m, 2H); 1.73-2.16 (m, 7H); 1.18-1.72 (m, 9H); 1.15 (s, 3H); 1.13 (s, 3H); 1.07 (d, 3H, J=7.5 Hz); 0.88 (d, 3H, J=7.5 Hz); 0.83 (t, 3H; J=7.5 Hz).
The diagnostic olefinic signal of the "1,4-reduction" side product appears as a multiplet at δ 5.42-5.49.

### EXAMPLE 2

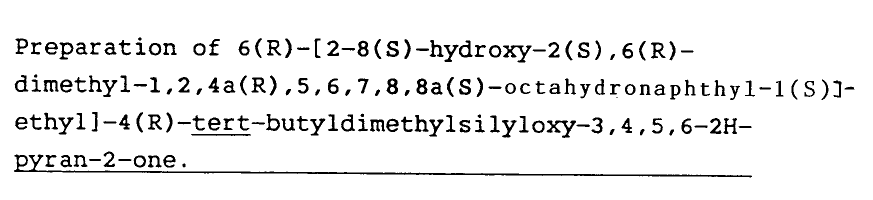

A solution of 6(R)-[2-[8(S)-hydroxy-2(S),6(R)-dimethyl-1,2,6,7,8,8a(R) hexahydronaphthyl-1(S)]-ethyl]-4(R)-tert-butyldimethylsilyloxy-3,4,5,6-2H-pyran-2-one (1.36 g, 3.12 mmol) in anhydrous dichloromethane (4.2 ml) and isopropanol (0.72 ml, 9.36 mmol) was briefly purged with argon gas. 1,5-Cyclooctadiene(pyridine)(tricyclohexylphosphine) iridium(I) hexafluorophosphate (62.5 mg, 2.5 mole percent) was added and the mixture reduced at atmospheric hydrogen pressure at ambient temperature for 21 hours.

The volatiles were removed in vacuo and the resulting solid taken up in 150 ml of warm diethyl ether, filtered (by vacuum) through a one inch bed of Florisil® (Magnesium silicate filter aid used herein to retain any catalyst complex which remained in solution) and washed with 2 x 50 ml of warm diethyl ether. The volatiles were again evaporated in vacuo to give a white crystalline solid which was identified by NMR as a 9:1 mixture of the title compound to the trans tetrahydro derivative. ¹HNMR (300 MHz, CDCl₃) δ 5.62 (ddd, J=2.5, 4.9, 9.5 Hz, 1H), 5.38 (d, J=9.5 Hz, 1H), 4.67 (m, 1H), 4.29 (m, 1H), 4.17 (m, 1H), 1.0-2.1 (m, 11H), 0.85 (m, 12H), 0.06 (s, 6H).

### EXAMPLE 3

### Preparation of 6(R)-[2-[8(S)-(2-methylbutyryloxy)-2(S),6(R)-dimethyl-1,2,3,5,6,7,8,8a(S)-octahydronaphthyl-1(S)]ethyl]-4(R)-hydroxy-3,4,5,6-tetrahydro-2H-pyran-2-one

A solution of lovastatin (0.63 g, 1.56 mmole) in dichloromethane (15 ml) was purged with argon gas. 1,5-Cyclooctadiene(pyridine)(tricyclohexylphosphine)iridium(I) hexafluorophosphate (0.0313 g, 2.5 mole percent) was added and the solution reduced at ambient temperature under one atmosphere hydrogen pressure. H₂ uptake was very rapid in the first 50 minutes. After 1 hour and 37 minutes, the reaction was stopped and the solution evaporated in vacuo to a yellow oil which was taken up in diethyl ether (50 ml) and filtered through a one inch bed of Florisil® (Magnesium silicate filter aid used herein to retain any catalyst complex which remained in solution). The filtrate was evaporated to give the title compound as a pale yellow oil. ¹HNMR (300 MHz, CDCl₃) δ 5.48 (brs, 1H), 5.28 (m, 1H) 4.63 (m, 1H), 4.35 (m, 1H), 2.74 (dd, J=16,7Hz, 1H), 2.61 (dd, J=16, 4Hz, 1H), 1.22-2.45(m), 1.12 (d, J=7Hz, 3H), 1.0 (d, J=7Hz, 3H), 0.87 (t, J=7Hz, 3H), 0.82 (d, J=7Hz, 3H).

### EXAMPLE 4

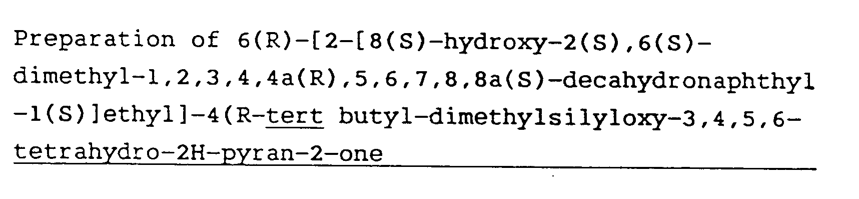

A solution of 6(R)-[2-[8(S)-hydroxy-2(S),6(R)-dimethyl-1,2,3,4,4a(R),5,6,7,8,8a(R)-hexahydronaphthyl-1(S)]ethyl]-4(R-tert butyl-dimethylsilyloxy-3,4,5,6-tetrahydro-2H-pyran-2-one (1.365 g, 3.13 mmol) in dichloromethane (15 ml) was purged briefly with argon gas. [Ir(COD)(PCy₃)(pyr)]PF₆ (62.5 mg, 2.5 mole percent) was added and the solution reduced at ambient temperature under 2.76x10⁵ Nm⁻² (40 psi) of hydrogen pressure for 24 hours. The solution was evaporated in vacuo to a solid which was taken up in 50 ml of warm diethyl ether, filtered through a one inch bed of Florisil® (Magnesium silicate filter aid used to retain any catalyst complex which remained in solution), washed with 45 ml of warm diethyl ether and evaporated in vacuo to give the title compound as a pale cream-colored solid. ¹H NMR (300 MHz, CDCl₃), δ 4.66 (m, 1H), 4.28 (m, 1H), 4.08 (m, 1H), 2.47-2.68 (m, 2H), 0.92-2.14 (m), 0.87 (s 9H), 0.82 (d J=2Hz, 3H), 0.07 (s, 3H), 0.05 (s, 3H).

## Claims

1. A process for the preparation of a compound of structural formula (I): 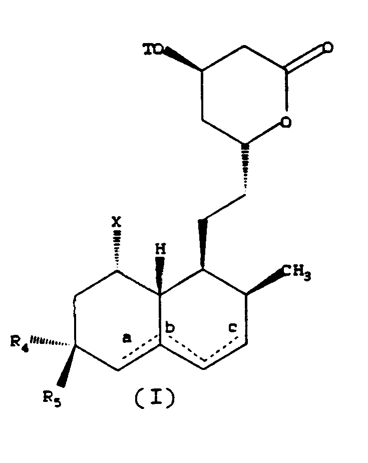 wherein:
T is H or t-C₄H₉(CH₃)₂Si;
X is 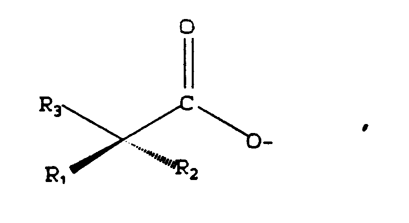 OH, NH₂, or OR₆;
R₁ is H or C₁₋₃alkyl;
R₂ is H or C₁₋₃alkyl;
R₃ is C₁₋₅alkyl, phenyl, or C₃₋₇ cycloalkyl; or C₁₋₅alkyl or phenyl substituted with a group Y where Y is selected from:
a) OH or t-C₄H₉(Me)₂SiO;
b) halogen;
c) trifluoromethyl;
d) C₁₋₃alkoxy;
e) C₁₋₃alkylcarbonyloxy;
f) phenylcarbonyloxy;
g) C₁₋₃alkoxycarbonyl; or
h) phenyloxycarbonyl;
R₄ is H, CH₃, CH₂OH, OH, CH₂OSi(Me)₂t-C₄H₉ or OSi(Me)₂t-C₄H₉;
R₅ is H, CH₂OH, OH, CH₂OSi(Me)₂t-C₄H₉ or OSi(Me)₂t-C₄H₉; provided that when either R₄ or R₅ is CH₂OH or CH₂OSi(Me)₂t-C₄H₉ the other is H; and one and only one of R₄ and R₅ can be OH or OSi(Me)₂t-C₄H₉;
R₆ is C₁₋₅alkyl, C₃₋₇ cycloalkyl, phenylC₁₋₃alkyl, or substituted phenylC₁₋₃alkyl in which the substituent is halogen, CN or CF₃;
a, b, c are double bonds or single bonds provided that only one of a, b or c is a double bond;
which comprises: contacting a compound of structural formula (II): 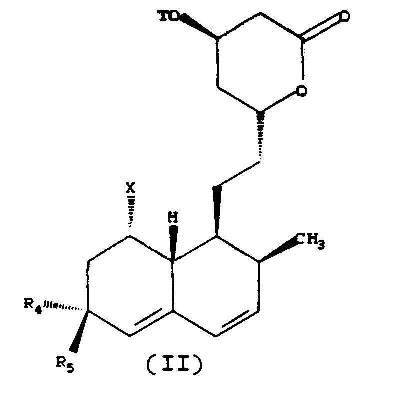 with a homogenous metal catalyst, in an organic solvent under a pressure of hydrogen gas at a temperature of 25-80°C provided that where a is a double bond:
X is 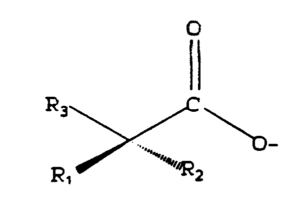
T is H; and
the homogenous metal catalyst is Rh(Ar₃P)₃ Z,
wherein
Ar is phenyl or naphthyl or C₁₋₃alkoxyphenyl or C₁₋₃alkoxynapthyl;
Z is Cl or Br;
the organic solvent is an alcoholic solvent; and the pressure is 2.76x10⁵-9.65x10⁶ Nm⁻² (40-1400 psi) at a temperature of 40-80°C; and
where b is a double bond:
X is 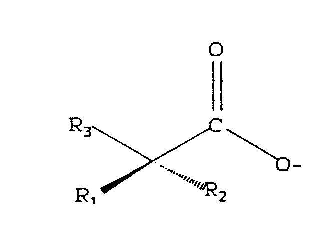
T is H; and
the homogenous metal catalyst is 1,5-cyclooctadiene(pyridine)(tricyclohexyl phosphine)iridium(I)hexafluorophosphate or norbornadiene-1,4-bis-(diphenylphosphino)butane-rhodium(I)-tetrafluoroborate;
the organic solvent is selected from dichloromethane, chloroform or chlorobenzene; and the pressure is 1.013x10⁵ Nm⁻² (one atmosphere) hydrogen at a temperature of 25 to 80°C; and
where c is a double bond:
X is OH, NH₂ or OR₆;
T is t-C₄H₉(CH₃)₂Si; the homogenous metal catalyst is 1,5-cyclooctadiene(pyridine)(tricyclohexyl phosphine)iridium(I)hexafluorophosphate or norbornadiene-1,4-bis-(diphenylphosphino)butane-rhodium(I)-tetrafluoroborate;
the organic solvent is an alcoholic solvent; and the pressure is one atmosphere hydrogen at ambient temperature; and
where a, b and c are all single bonds:
X is OH, NH₂ or OR₆;
T is t-C₄H₉(CH₃)₂Si;
the homogenous metal catalyst is 1,5-cyclooctadiene(pyridine)(tricyclohexylphosphine)iridium(I)hexafluorophosphate or norbornadiene-1,4-bis-(diphenylphosphino)butane-rhodium(I)-tetrafluoroborate;
the organic solvent is selected from dichloromethane or chloroform, or chlorobenzene; the pressure is 1.03x10⁵-2.76x10⁵ Nm⁻² (15-40 psi) at a temperature of about 25-80°C.

2. A process according to Claim 1 wherein the compound prepared is of formula (Ia): 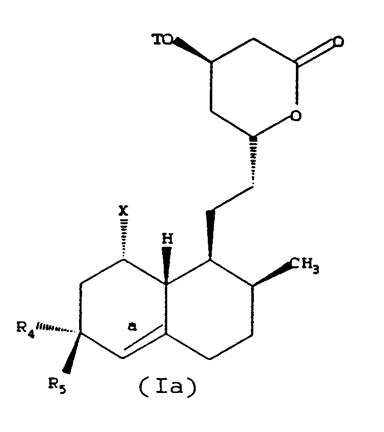 wherein:
T is H;
X is 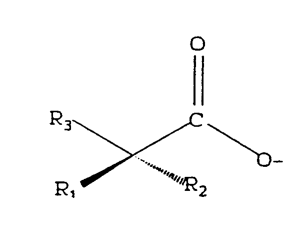
R₁ is methyl; the homogenous metal catalyst is Rh(Ar₃P)₃Z;
R₃ is CH₃CH₂;
R₂, R₄ and R₅ are as defined in claim 1;
Ar is phenyl or naphthyl or C₁₋₃ alkoxy substituted phenyl or naphthyl;
Z is Cl or Br;
the organic solvent is isopropanol or ethanol;
the hydrogen gas is at a pressure of 4.14x10⁶-9.65x10⁶ Nm⁻² (600-1400 psi); at a temperature of about 40°C.

3. A process according to Claim 2 wherein:
The solvent is isopropanol;
The catalyst is tris(triphenylphosphine) rhodium (I) chloride at a weight percent ratio of 5 percent catalyst to substrate (II) and the compound I(a) prepared is selected from the group wherein:
a. R₂ is CH₃, R₄ is CH₃, R₅ is H;
b. R₂ is H, R₄ is CH₃, R₅ is H;
c. R₂ is CH₃, R₄ is CH₂OH, R₅ is H;
d. R₂ is CH₃, R₄ is H, R₅ is CH₂OH;
e. R₂ is H, R₄ is CH₂OH, R₅ is H;
f. R₂ is H, R₄ is H, R₅ is CH₂OH;
g. R₂ is CH₃, R₄ is CH₂OSi(Me)₂t-C₄H₉, R₅ is H;
h. R₂ is CH₃, R₄ is H, R₅ is CH₂OSi(Me)₂t-C₄H₉.

4. A process according to Claim 1 wherein the compound prepared is of formula I(c): 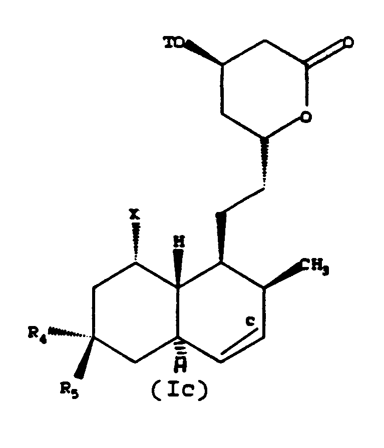 wherein:
T is t-C₄H₉(Me)₂Si;
X is OH, NH₂, or OR₆; wherein R₆ is C₁₋₅ alkyl, C₃₋₇ cycloalkyl, phenylC₁₋₃alkyl, or substituted phenylC₁₋₃alkyl in which the substituent is halogen, CF₃ or CN;
R₄ is H, CH₃, CH₂OSi(Me)₂t-C₄H₉ or OSi(Me)₂t-C₄H₉;
R₅ is H, CH₂OSi(Me)₂t-C₄H₉ or OSi(Me)₂t-C₄H₉; provided that when one of R₄ and R₅ is CH₂OSi(Me)₂t-C₄H₉ the other is H;
the homogenus metal catalyst is 1,5-cyclooctadiene(pyridine)(tricyclohexylphosphine)iridium (I) hexafluorophospate or norbornadiene-1,4-bis(diphenylphosphino) butane rhodium (I) hexafluoroborate; the organic solvent is a mixture of dichloromethane, chloroform or chlorobenzene and ethanol or isopropanol.

5. A process according to Claim 4 wherein:
X is OH;
the catalyst is 1,5-cyclooctadiene(pyridine) (tricyclohexylphosphine)iridium (I) hexafluorophospate;
the solvent is a mixture of dichloromethane, and isopropanol;
and the compound I(c) prepared is selected from the group wherein:
a. R₄ is CH₃, R₅ is H;
b. R₄ is CH₂OSi(Me)₂t-C₄H₉, R₅ is H;
c. R₄ is H, R₅ is CH₂OSi(Me)₂t-C₄H₉;
d. R₄ is OSi(Me)₂t-C₄H₉, R₅ is H;
e. R₄ is H, R₅ is OSi(Me)₂t-C₄H₉;
f. R₄ is CH₃, R₅ is OSi(Me)₂t-C₄H₉.

6. A process according to Claim 1 wherein the compound prepared is of formula I(b): 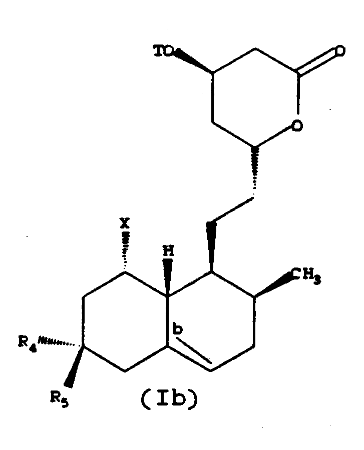 wherein:
T is H;
X is 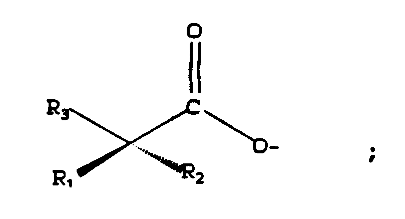
R₁ is CH₃;
R₂ is H or C₁₋₃alkyl;
R₃ is C₁₋₅alkyl, phenyl, or C₃₋₇ cycloalkyl; or C₁₋₅alkyl or phenyl substituted with a group Y where Y is a group not reduced by the catalyst herein, examples of such a group Y are:
a) t-C₄H₉(Me)₂SiO-;
b) halogen (F, Cl or Br);
c) trifluoromethyl;
d) C₁₋₃ alkoxy;
e) C₁₋₃alkylcarbonyloxy;
f) phenylcarbonyloxy;
g) C₁₋₃alkoxycarbonyl; or
h) phenyloxycarbonyl;
R₄ is H, CH₃, CH₂OSi(Me)₂t-C₄H₉ or OSi(Me)₂t-C₄H₉;
R₅ is H, CH₂OSi(Me)₂t-C₄H₉ or OSi(Me)₂t-C₄H₉; provided that when either of R₄ and R₅ is CH₂OSi(Me)₂t-C₄H₉ the other is H;
the homogenous metal catalyst is 1,5-cyclooctadiene(pyridine)(tricyclohexylphosphine)iridium (I), hexafluorophosphate or norbornadiene-1,4-bis(diphenylphosphino)-butane-rhodium (I)-hexafluoroborate;
the organic solvent is dichloromethane, chloroform or chlorobenzene; at a temperature of about 25°C.

7. A process according to Claim 6 wherein:
R₃ is CH₃CH₂;
R₄ is H, CH₃ or CH₂OSi(Me)₂t-C₄H₉;
R₅ is H or CH₂OSi(Me)₂t-C₄H₉;
provided that at least one of R₄ or R₅ is H;
the catalyst is 1,5-cyclooctadiene (pyridine) (tricyclohexylphosphine) iridium (I) hexafluorophosphate;
the solvent is dichloromethane;
the mole percent catalyst to diene is 2.5 percent;
and the compound I(b) prepared is selected from the group wherein:
a. R₂ is CH₃, R₄ is CH₃, R₅ is H;
b. R₂ is H, R₄ is CH₃, R₅ is H;
c. R₂ is CH₃, R₄ is CH₂OSi(Me)₂t-C₄H₉, R₅ is H;
d. R₂ is CH₃, R₄ is H, R₅ is CH₂OSi(Me)₂t-C₄H₉;
e. R₂ is H, R₄ is CH₂OSi(Me)₂t-C₄H₉, R₅ is H;
f. R₂ is H, R₄ is H, R₅ is CH₂OSi(Me)₂t-C₄H₉.

8. A process according the Claim 1 wherein the compound prepared is of formula I(d): 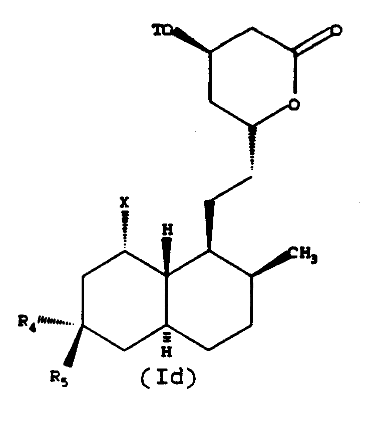 wherein:
T is t-C₄H₉(CH₃)₂Si;
X is OH, NH₂ or OR₆; wherein R₆ is C₁₋₅ alkyl, C₃₋₇ cycloalkyl, phenyl-C₁₋₃ alkyl, or substituted phenyl-C₁₋₃ alkyl in which the substituent is halogen, CF₃ or CN;
R₄ is H, CH₃, CH₂OSi(Me)₂t-C₄H₉ or OSi(Me)₂t-C₄H₉;
R₅ is H, CH₂OSi(Me)₂t-C₄H₉ or OSi(Me)₂t-C₄H₉;
provided that when one of R₄ and R₅ is CH₂OSi(Me)₂t-C₄H₉ the other is H;
the homogenous metal catalyst is 1,5-cyclooctadiene(pyridine)(tricyclohexylphosphine)iridium(I), hexafluorophosphate or norbornadiene-1,4-bis(diphenylphosphino)-butane-rhodium(I)-hexafluoroborate;
the organic solvent is dichloromethane, chloroform or chlorobenzene; at a temperature of about 25°C.

9. A process according the Claim 8 wherein:
X is OH; the solvent is dichloromethane; the catalyst is 1,5-cyclooctadiene (tricyclohexylphosphine) (pyridine) iridium (I)-hexafluorophosphate;
the hydrogen gas is at a pressure of 2.76x10⁵ Nm⁻² (40 psi);
the temperature is about 25°C;
and the compound I(d) prepared is selected from the group wherein:
a. R₄ is CH₃, R₅ is H;
b. R₄ is CH₂OSi(Me)₂t-C₄H₉, R₅ is H;
c. R₄ is H, R₅ is CH₂OSi(Me)₂t-C₄H₉;
d. R₄ is OSi(Me)₂t-C₄H₉, R₅ is H;
e. R₄ is H, R₅ is OSi(Me)₂t-C₄H₉;
f. R₄ is CH₃, R₅ is OSi(Me)₂t-C₄H₉.

## Patentansprüche

1. Verfahren zur Herstellung einer Verbindung mit der Strukturformel (I): 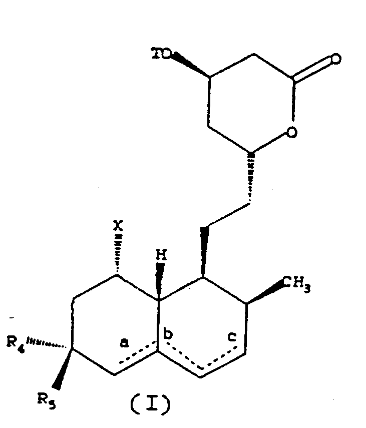 worin :
T H oder t-C₄H₉(CH₃)₂Si bedeutet;
X 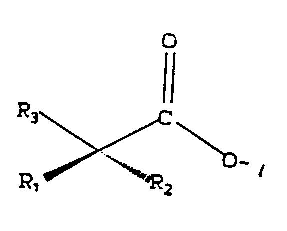 OH, NH₂ oder OR₆ darstellt;
R₁ für H oder C₁₋₃-Alkyl steht;
R₂ H oder C₁₋₃-Alkyl darstellt;
R₃ C₁₋₅-Alkyl, Phenyl oder C₃₋₇-Cycloalkyl; oder
C₁₋₅-Alkyl oder Phenyl, substituiert mit einer Gruppe Y, bedeutet, worin Y ausgewählt wird aus:
a) OH oder t-C₄H₉(Me)₂SiO;
b) Halogen;
c) Trifluormethyl;
d) C₁₋₃-Alkoxy;
e) C₁₋₃-Alkylcarbonyloxy;
f) Phenylcarbonyloxy;
g) C₁₋₃-Alkoxycarbonyl; oder
h) Phenyloxycarbonyl;
R₄ H, CH₃, CH₂OH, OH, CH₂OSi(Me)₂t-C₄H₉ oder OSi(Me)₂t-C₄H₉ bedeutet
R₅ für H, CH₂OH, OH, CH₂OSi(Me)₂t-C₄H₉ oder OSi(Me)₂t-C₄H₉ steht, mit der Maßgabe, daß, wenn entweder R₄ oder R₅ CH₂OH oder CH₂OSi(Me)₂t-C₄H₉ bedeutet, das andere H darstellt und eines und nur eines von R₄ und R₅ OH oder OSi(Me)₂t-C₄H₉ sein kann;
R₆ C₁₋₅-Alkyl, C₃₋₇-Cycloalkyl, Phenyl-C₁₋₃-alkyl oder substituiertes Phenyl-C₁₋₃-alkyl, worin der Substituent Halogen, CN oder CF₃ ist, darstellt;
a, b, c für Doppelbindungen oder Einzelbindungen stehen, mit der Maßgabe, daß nur eines von a, b oder c eine Doppelbindung darstellt;
welches umfaßt:
Kontaktieren einer Verbindung der Strukturformel (II): 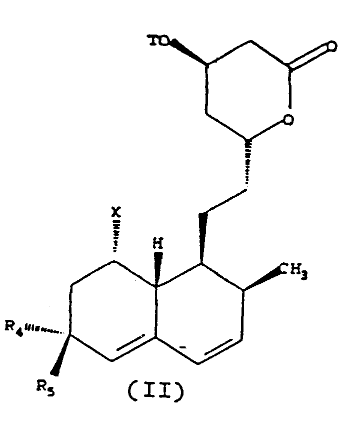 mit einem homogenen Metallkatalysator in einem organischen Lösungsmittel unter einem Druck von Wasserstoffgas bei einer Temperatur von 25-80 °C, mit der Maßgabe, daß, wo a eine Doppelbindung ist:
X für 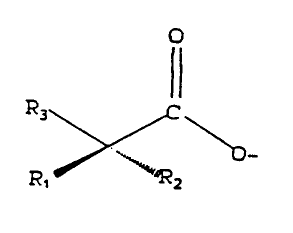 steht;
T H bedeutet; und
der homogene Metallkatalysator Rh(Ar₃P)₃Z ist, worin
Ar für Phenyl oder Naphthyl oder C₁₋₃-Alkoxyphenyl oder C₁₋₃-Alkoxynaphthyl steht;
Z Cl oder Br bedeutet;
das organische Lösungsmittel ein alkoholisches Lösungsmittel ist; und der Druck 2,76x10⁵ - 9,65x10⁶ Nm⁻² (40-1400 psi) bei einer Temperatur von 40-80 °C beträgt; und
wo b eine Doppelbindung ist:
X 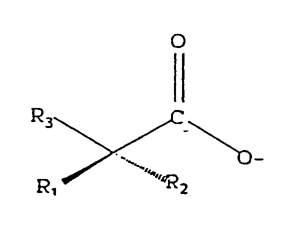 darstellt;
T H bedeutet; und
der homogene Metallkatalysator 1,5-Cyclooctadien-(pyridin)-(tricyclohexylphosphin)-iridium(I)-hexafluorphosphat oder Norbornadien-1,4-bis(diphenylphosphino)-butanrhodium(I)-tetrafluorborat ist;
das organische Lösungsmittel ausgewählt wird aus Dichlormethan, Chloroform oder Chlorbenzol; und der Druck 1,013x10⁵ Nm⁻² (eine Atmosphäre) Wasserstoff bei einer Temperatur von 25-80 °C beträgt; und
wo c eine Doppelbindung ist:
X für OH, NH₂ oder OR₆ steht;
T t-C₄H₉(CH₃)₂Si bedeutet;
der homogene Metallkatalysator 1,5-Cyclooctadien-(pyridin)-(tricyclohexylphosphin)-iridium(I)-hexafluorphosphat oder Norbornadien-1,4-bis(diphenylphosphino)-butanrhodium(I)-tetrafluorborat ist;
das organische Lösungsmittel ein alkoholisches Lösungsmittel darstellt; und der Druck eine Atmosphäre Wasserstoff bei Umgebungstemperatur beträgt; und
wo a, b und c alle Einzelbindungen sind:
X für OH, NH₂ oder OR₆ steht;
T t-C₄H₉(CH₃)₂Si bedeutet;
der homogene Metallkatalysator 1,5-Cyclooctadien-(pyridin)-(tricyclohexylphosphin)-iridium(I)-hexafluorphosphat oder Norbornadien-1,4-bis(diphenylphosphino)-butanrhodium(I)-tetrafluorborat ist;
das organische Lösungsmittel ausgewählt wird aus Dichlormethan oder Chloroform oder Chlorbenzol; der Druck 1,03x10⁵ - 2,76x10⁵ Nm⁻² (15-40 psi) bei einer Temperatur von etwa 25-80 °C beträgt.

2. Verfahren nach Anspruch 1, bei dem die hergestellte Verbindung die Formel (Ia): 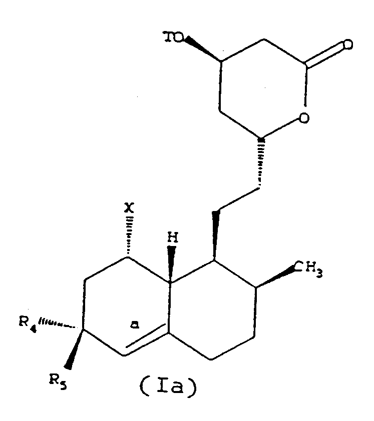 besitzt;
worin:
T für H steht;
X 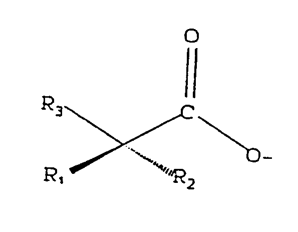 bedeutet;
R₁ für Methyl steht;
der homogene Metallkatalysator Rh(Ar₃P)₃Z darstellt;
R₃ CH₃CH₂ bedeutet;
R2, R4 und R5 die in Anspruch 1 gegebene Definition besitzen;
Ar Phenyl oder Naphthyl oder C₁₋₃-Alkoxy, substituiertes Phenyl oder Naphthyl darstellt;
Z für Cl oder Br steht;.
das organische Lösungsmittel Isopropanol oder Ethanol ist;
das Wasserstoffgas bei einem Druck von 4,14x10⁶ - 9,65x10⁶ Nm⁻² (600-1400 psi) vorliegt; bei einer Temperatur von etwa 40 °C.

3. Verfahren nach Anspruch 2 bei dem:
das Lösungsmittel Isopropanol ist;
der Katalysator Tris(triphenylphosphin)-rhodium(I)-chlorid ist, mit einem Gewichtsprozentverhältnis Katalysator zu Substrat (II) von 5 Prozent, und die hergestellte Verbindung I(a) ausgewählt wird aus der Gruppe, in der:
a. R₂ CH₃ bedeutet, R₄ CH₃ darstellt, R₅ für H steht;
b. R₂ H bedeutet, R₄ CH₃ darstellt, R₅ für H steht;
c. R₂ CH₃ bedeutet, R₄ CH₂OH darstellt, R₅ für H steht;
d. R₂ CH₃ bedeutet, R₄ H darstellt, R₅ für CH₂OH steht;
e. R₂ H bedeutet, R₄ CH₂OH darstellt, R₅ für H steht;
f. R₂ H bedeutet, R₄ H darstellt, R₅ für CH₂OH steht;
g. R₂ CH₃ bedeutet, R₄ CH₂OSi(Me)₂t-C₄H₉ darstellt, R₅ für H steht;
h. R₂ CH₃ bedeutet, R4 H darstellt, R₅ für CH₂OSi(Me)₂t-C₄H₉ steht.

4. Verfahren nach Anspruch 1, bei dem die hergestellte Verbindung die Formel I(c): 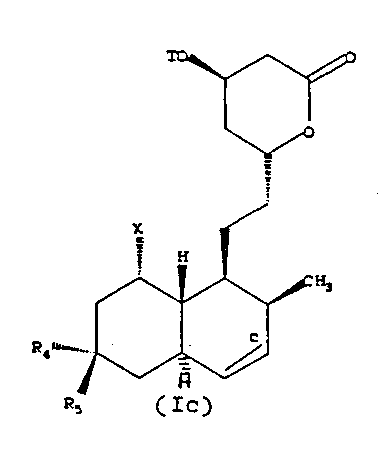 besitzt;
worin:
T für t-C₄H₉(Me)₂Si steht,
X OH, NH₂ oder OR₆ bedeutet, worin R₆ für C₁₋₅-Alkyl, C₃₋₇-Cycloalkyl, Phenyl-C₁₋₃-alkyl oder substituiertes Phenyl-C₁₋₃-alkyl, worin der Substituent Halogen, CF₃ oder CN darstellt, steht;
R₄ H, CH₃, CH₂OSi(Me)₂t-C₄H₉ oder OSi(Me)₂t-C₄H₉ bedeutet;
R₅ H, CH₂OSi(Me)₂t-C₄H₉ oder OSi(Me)₂t-C₄H₉ bedeutet, mit der Maßgabe, daß, wenn eines von R₄ und R₅ CH₂OSi(Me)₂t-C₄H₉ ist, das andere für H steht; der homogene Metallkatalysator 1,5-Cyclooctadien-(pyridin)-(tricyclohexylphosphin)-iridium(I)-hexafluorphosphat oder Norbornadien-1,4-(diphenylphosphino)-butanrhodium(I)- hexafluorborat ist;
das organische Lösungsmittel ein Gemisch aus Dichlormethan, Chloroform oder Chlorbenzol und Ethanol oder Isopropanol ist.

5. Verfahren nach Anspruch 4, bei dem:
X für OH steht;
der Katalysator 1,5-Cyclooctadien-(pyridin)-(tricyclohexylphosphin)-iridium(I)-hexafluorphosphat ist;
das Lösungsmittel ein Gemisch aus Dichlormethan und Isopropanol darstellt;
und die hergestellte Verbindung I(c) ausgewählt wird aus der Gruppe, in der:
a. R₄ CH₃ bedeutet, R₅ H darstellt;
b. R₄ CH₂OSi(Me)₂t-C₄H₉ bedeutet, R₅ H darstellt;
c. R₄ H bedeutet, R₅ CH₂OSi(Me)₂t-C₄H₉ darstellt;
d. R₄ OSi(Me)₂t-C₄H₉ bedeutet, R₅ H darstellt;
e. R₄ H bedeutet, R₅ OSi(Me)₂t-C₄H₉ darstellt;
f. R₄ CH₃ bedeutet, R₅ OSi(Me)₂t-C₄H₉ darstellt.

6. Verfahren nach Anspruch 1, bei dem die hergestellte Verbindung die Formel I(b) besitzt: 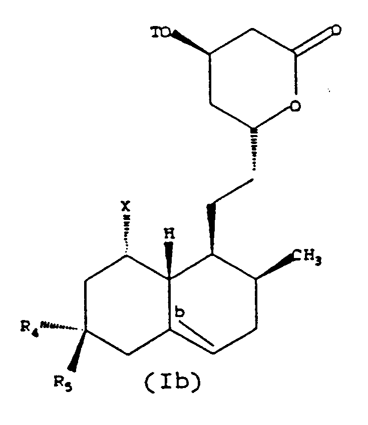 worin:
T für H steht;
X 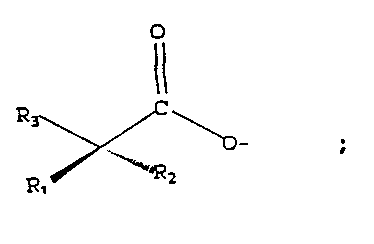 bedeutet;
R₁ für CH₃ steht;
R₂ H oder C₁₋₃-Alkyl bedeutet;
R₃ C₁₋₅-Alkyl, Phenyl oder C₃₋₇-Cycloalkyl; oder
C₁₋₅-Alkyl oder Phenyl, substituiert mit einer Gruppe Y, bedeutet, worin Y eine Gruppe ist, die nicht durch den Katalysator hierin reduziert wird, wobei Beispiele für eine solche Gruppe Y folgende sind:
a) t-C₄H₉(Me)₂SiO-;
b) Halogen (F, Cl oder Br);
c) Trifluormethyl;
d) C₁₋₃-Alkoxy;
e) C₁₋₃-Alkylcarbonyloxy;
f) Phenylcarbonyloxy;
g) C₁₋₃-Alkoxycarbonyl; oder
h) Phenyloxycarbonyl;
R₄ für H, CH₃, CH₂OSi(Me)₂t-C₄H₉ oder OSi(Me)₂t-C₄H₉ steht;
R₅ H, CH₂OSi(Me)₂t-C₄H₉ oder OSi(Me)₂t-C₄H₉ bedeutet, mit der Maßgabe, daß, wenn eines von R₄ und R₅ CH₂OSi(Me)₂t-C₄H₉OH bedeutet, das andere für H steht;
der homogene Metallkatalysator 1,5-Cyclooctadien-(pyridin)-(tricyclohexylphosphin)-iridium(I)-hexafluorphosphat oder Norbornadien-1,4-bis(diphenylphosphino)-butanrhodium(I)- hexafluorborat ist;
das organische Lösungsmittel Dichlormethan, Chloroform oder Chlorbenzol darstellt; bei einer Temperatur von etwa 25 °C.

7. Verfahren nach Anspruch 6, bei dem:
R₃ für CH₃CH₂ steht;
R₄ H, CH₃ oder CH₂OSi(Me)₂t-C₄H₉ darstellt;
R₅ für H oder CH₂OSi(Me)₂t-C₄H₉ steht;
mit der Maßgabe, daß mindestens eines von R₄ oder R₅ für H steht;
der Katalysator 1,5-Cyclooctadien-(pyridin)-(tricyclohexylphosphin)-iridium(I)-hexafluorphosphat ist;
das Lösungsmittel Dichlormethan ist;
das Molprozentverhältnis von Katalysator zu Dien 2,5 Prozent beträgt;
und die hergestellte Verbindung I(b) ausgewählt wird aus der Gruppe, in der:
a. R₂ CH₃ bedeutet, R₄ CH₃ darstellt, R₅ für H steht;
b. R₂ H bedeutet, R₄ CH₃ darstellt, R₅ für H steht;
c. R₂ CH₃ bedeutet, R₄ CH₂OSi(Me)₂t-C₄H₉ darstellt, R₅ für H steht;
d. R₂ CH₃ bedeutet, R₄ H darstellt, R₅ für CH₂OSi(Me)₂t-C₄H₉ steht;
e. R₂ H bedeutet, R₄ CH₂OSi(Me)₂t-C₄H₉ darstellt, R₅ für H steht;
f. R₂ H bedeutet, R₄ H darstellt, R₅ für CH₂OSi(Me)₂t-C₄H₉ steht.

8. Verfahren nach Anspruch 1, bei dem die hergestellte Verbindung die Formel I(d) besitzt: 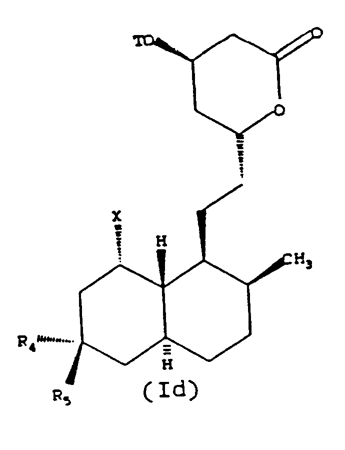 worin:
T für t-C₄H₉(CH₃)₂Si steht;
X OH, NH₂ oder OR₆ bedeutet, worin R₆ C₁₋₅-Alkyl, C₃₋₇-Cycloalkyl, Phenyl-C₁₋₃-alkyl oder substituiertes Phenyl-C₁₋₃-alkyl, worin der Substituent Halogen, CF₃ oder CN ist, darstellt;
R₄ für H, CH₃, CH₂OSi(Me)₂t-C₄H₉ oder OSi(Me)₂t-C₄H₉ steht;
R₅ H, CH₂OSi(Me)₂t-C₄H₉ oder OSi(Me)₂t-C₄H₉ bedeutet; mit der Maßgabe, daß, wenn eines von R₄ und R₅ CH₂OSi(Me)₂t-C₄H₉ ist, das andere für H steht; der homogene Metallkatalysator 1,5-Cyclooctadien-(pyridin)-(tricyclohexylphosphin)-iridium(I)-hexafluorphosphat oder Norbornadien-1,4-bis(diphenylphosphino)-butanrhodium(I)-hexafluorborat darstellt;
das organische Lösungsmittel Dichlormethan, Chloroform oder chlorbenzol ist; bei einer Temperatur von etwa 25 °C.

9. Verfahren nach Anspruch 8, bei dem:
X für OH steht;
das Lösungsmittel Dichlormethan ist;
der Katalysator 1,5-Cyclooctadien-(tricyclohexylphosphin)-(pyridin)-iridium(I)-hexafluorphosphat ist;
das Wasserstoffgas bei einem Druck von 2,76x10⁵ Nm⁻² (40 psi) vorliegt;
die Temperatur etwa 25 °C beträgt;
und die hergestellte Verbindung I(d) ausgewählt wird aus der Gruppe, worin:
a. R₄ CH₃ bedeutet, R₅ H darstellt;
b. R₄ CH₂OSi(Me)₂t-C₄H₉ bedeutet, R₅ H darstellt;
c. R₄ H bedeutet, R₅ CH₂OSi(Me)₂t-C₄H₉ darstellt;
d. R₄ OSi(Me)₂t-C₄H₉ bedeutet, R₅ H darstellt;
e. R₄ H bedeutet, R₅ OSi(Me)₂t-C₄H₉ darstellt;
f. R₄ CH₃ bedeutet, R₅ OSi(Me)₂t-C₄H₉ darstellt.

## Revendications

1. Procédé de préparation d'un composé de formule structurale (I) : 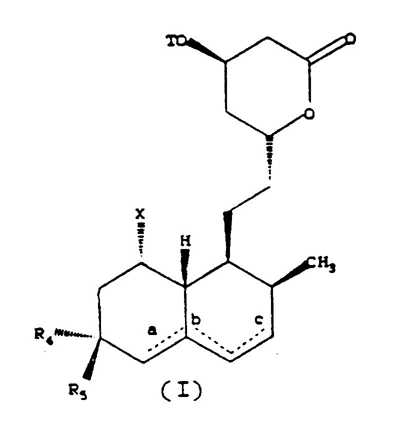 dans laquelle :
T est H ou t-C₄H₉-(CH₃)₂Si;
X est 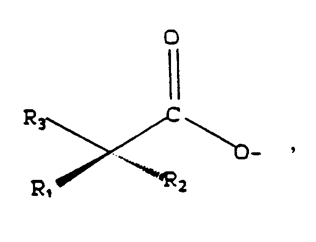 OH, NH₂, ou OR₆;
R₁ est H ou alkyle C₁₋₃;
R₂ est H ou alkyle C₁₋₃;
R₃ est alkyle C₁₋₅, phényle, ou cycloalkyle C₃₋₇; ou alkyle C₁₋₅ ou phényle substitué par un groupe Y, Y étant sélectionné parmi :
a) OH ou t-C₄H₉(Me)₂SiO;
b) halogène;
c) trifluorométhyle;
d) alcoxy C₁₋₃;
e) (alkyl C₁₋₃)carbonyloxy;
f) phénylcarbonyloxy;
g) (alcoxy C₁₋₃)carbonyle; ou
h) phényloxycarbonyle;
R₄ est H, CH₃, CH₂OH, OH, CH₂OSi(Me)₂t-C₄H₉ ou OSi(Me)₂t-C₄H₉;
R₅ est H, CH₂OH, OH, CH₂OSi(Me)₂t-C₄H₉ ou OSi(Me)₂t-C₄H₉; pourvu que quand soit R₄ soit R₅ est CH₂OH ou CH₂OSi(Me)₂t-C₄H₉ l'autre est H; et un et un seul de R₄ et R₅ peut être OH ou OSi(Me)₂t-C₄H₉;
R₆ est un alkyle C₁₋₅, cycloalkyle C₃₋₇, phényl(alkyl C₁₋₃) ou un phényl(alkyl C₁₋₃) substitué dans lequel le substituant est un halogène, CN ou CF₃;
a, b, c sont des doubles liaisons ou des liaisons simples à la condition que seulement une parmi a, b ou c est une double liaison;
qui comprend :
la mise en contact d'un composé de formule structurale (II) : 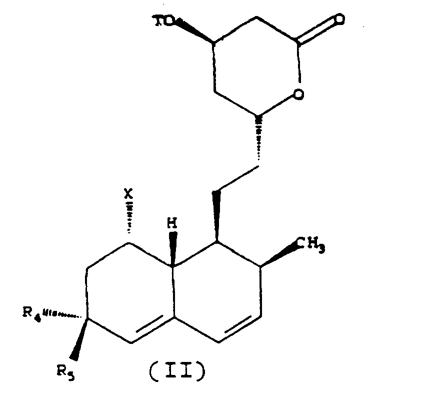 avec un catalyseur métallique homogène dans un solvant organique sous une pression de gaz hydrogène à une température de 25-80°C à la condition que là où a est une double liaison :
X est 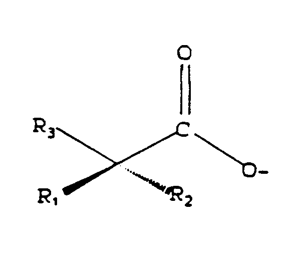
T est H; et
le catalyseur métallique homogène est Rh(Ar₃P)₃ Z dans laquelle
Ar est phényle ou naphtyle ou (alcoxy C₁₋₃)phényle ou (alcoxy C₁₋₃)alcoxynaphtyle;
Z est Cl ou Br;
le solvant organique est un solvant alcoolique ; et la pression est 2,76x10⁵-9,65x10⁶Nm⁻² (40-14000 psi) à une température de 40-80°C; et
quand b est une double liaison :
X est 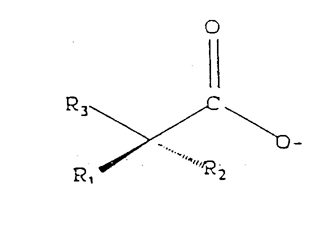
T est H ; et
le catalyseur métallique homogène est 1,5-cyclooctadiène(pyridine)(tricyclohexyl phosphine) iridium(I)hexafluorophosphate ou norbornadiène-1,4-bis-(diphénylphosphino)butane-rhodium(I)-tétrafluoroborate ;
le solvant organique est choisi parmi dichlorométhane, chloroforme ou 1,013x10⁵Nm⁻² chlorobenzène; et la pression est (une atmosphère) d'hydrogène à la température de 25 à 80°C ; et
quand c est une double liaison :
X est OH, NH₂ ou OR₆ ;
T est t-C₄H₉(CH₃)₂Si ;
le catalyseur métallique homogène est 1,5-cyclooctadiène(pyridine)(tricyclohexyl phosphine) iridium(I)hexafluorophosphate ou norbornadiène-1,4-bis-(diphénylphosphino)butane-rhodium(I)-tétrafluoroborate ;
le solvant organique est un solvant alcoolique; et la pression est de une atmosphère d'hydrogène à température ambiante ; et
quand a, b et c sont toutes des liaisons simples :
X est OH, NH₂ ou OR₆;
T est t-C₄H₉(CH₃)₂Si;
le catalyseur métallique homogène est 1,5-cyclooctadiène(pyridine)(tricyclohexylphosphine) iridium(I)hexafluorophosphate ou norbornadiène-1,4-bis-(diphénylphosphino)butane-rhodium(I)-tétrafluoroborate ;
le solvant organique est choisi parmi dichlorométhane ou chloroforme ou 1,03x10⁵-2,76x10⁵Nm⁻² chlorobenzène ; la pression est (15-40 psi) à une température d'environ 25-80°C.

2. Procédé selon la revendication 1, dans lequel le composé préparé a la formule (Ia) : 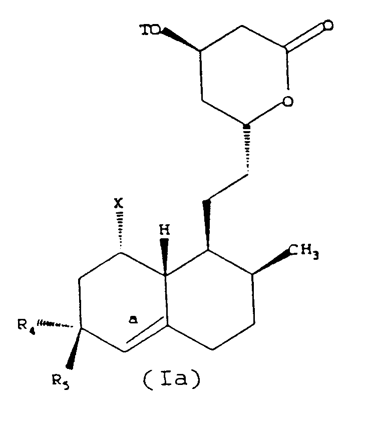 dans laquelle :
T est H ;
X est 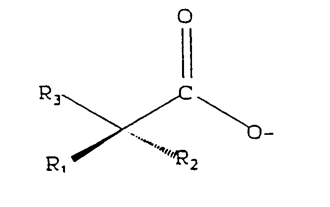
R₁ est un méthyle ; le catalyseur métallique homogène est Rh(Ar₃P)₃Z;
R₃ est CH₃CH₂ ;
R₂, R₄ et R₅ sont tels que définis à la revendication 1;
Ar est phényle ou naphtyle ou phényle ou naphtyle substitué par un (alcoxy C₁₋₃) ;
Z est Cl ou Br ;
le solvant organique est l'isopropanol ou l'éthanol ;
le gaz hydrogène est à une pression de 4,14x10⁶-9,65x10⁶Nm⁻² (600-1400 psi) à une température d'environ 40°C.

3. Procédé selon la revendication 2 dans lequel: le solvant est l'isopropanol;
le catalyseur est le chlorure de tris(triphénylphosphine)rhodium (I) à une concentration en pourcentage pondéral de 5 pourcent de catalyseur par rapport au substrat (II) et le composé (Ia) préparé est choisi dans le groupe dans lequel :
a. R₂ est CH₃, R₄ est CH₃, R₅ est H;
b. R₂ est H, R₄ est CH₃, R₅ est H;
c. R₂ est CH₃, R₄ est CH₂OH, R₅ est H;
d. R₂ est CH₃, R₄ est H, R₅ est CH₂OH;
e. R₂ est H, R₄ est CH₂OH, R₅ est H;
f. R₂ est H, R₄ est H, R₅ est CH₂OH;
g. R₂ est CH₃, R₄ est CH₂OSi(Me)₂t-C₄H₉, R₅ est H;
h. R₂ est CH₃, R₄ est H, R₅ est CH₂OSi(Me)₂t-C₄H₉.

4. Procédé selon la revendication 1 dans lequel le produit préparé a la formule I(c) : 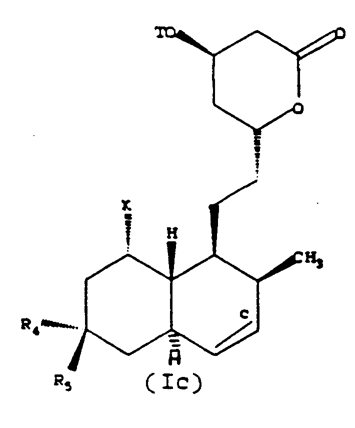 dans laquelle
T est t-C₄H₉(Me)₂Si;
X est OH, NH₂, ou OR₆; dans laquelle R₆ est alkyle C₁₋₅, cycloalkyle C₃₋₇, phényl(alkyle C₁₋₃), ou phényl(alkyle C₁₋₃) substitué dans laquelle le substituant est un halogène CF₃ ou CN;
R₄ est H, CH₃, CH₂OSi(Me)₂t-C₄H₉ ou OSi(Me)₂t-C₄H₉;
R₅ est H, CH₂OSi(Me)₂t-C₄H₉ ou OSi(Me)₂t-C₄H₉; à la condition que si l'un de R₄ et R₅ est CH₂OSi(Me)₂t-C₄H₉ l'autre est H;
le catalyseur métallique homogène est 1,5-cyclooctadiène(pyridine)(tricyclohexylphosphine) iridium (I) hexafluorophosphate ou norbornadiène-1,4-bis(diphénylphosphino)butane rhodium (I) hexafluoroborate;
le solvant organique est un mélange de dichlorométhane, chloroforme ou chlorobenzène et éthanol ou isopropanol.

5. Procédé selon la revendication 4 dans lequel:
X est OH;
le catalyseur est 1,5-cyclooctadiène(pyridine)(tricyclohexylphosphine) iridium (I) hexafluorophosphate;
le solvant est un mélange de dichlorométhane et d'isopropanol;
et le composé I(c) préparé est choisi dans le groupe dans lequel :
a. R₄ est CH₃, R₅ est H;
b. R₄ est CH₂OSi(Me)₂t-C₄H₉, R₅ est H;
c. R₄ est H, R₅ est CH₂OSi(Me)₂t-C₄H₉;
d. R₄ est OSi(Me)₂t-C₄H₉, R₅ est H;
e. R₄ est H, R₅ est OSi(Me)₂t-C₄H₉;
f. R₄ est CH₃, R₅ est OSi(Me)₂t-C₄H₉.

6. Procédé selon la revendication 1 dans lequel le composé préparé a la formule I(b) : 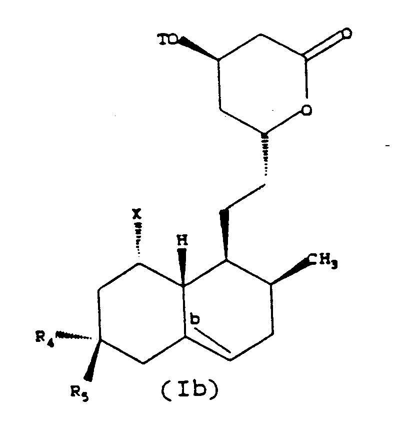 dans laquelle :
T est H;
X est 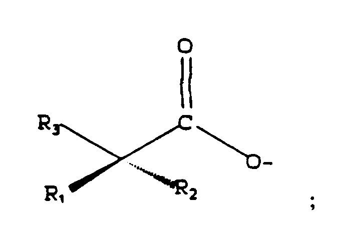
R₁ est CH₃;
R₂ est H ou alkyle C₁₋₃,
R₃ est alkyle C₁₋₅, phényle ou cycloalkyle C₃₋₇;
ou alkyle C₁₋₅ ou phényle substitué par un groupe Y, Y étant un groupe non réduit par le catalyseur utilisé ici, des exemples de tels groupes Y sont :
a) t-C₄H₉(Me)₂SiO-;
b) halogène (F, Cl ou Br);
c) trifluorométhyle;
d) alcoxy C₁₋₃;
e) (alkyle C₁₋₃)carbonyloxy;
f) phénylcarbonyloxy;
g) (alcoxy C₁₋₃)carbonyle; ou
h) phényloxycarbonyle;
R₄ est H, CH₃, CH₂OSi(Me)₂t-C₄H₉ ou OSi(Me)₂t-C₄H₉;
R₅ est H, CH₂OSi(Me)₂t-C₄H₉ ou
OSi(Me)₂t-C₄H₉; à condition que si l'un de R₄ et R₅ est CH₂OSi(Me)₂t-C₄H₉ l'autre est H;
le catalyseur métallique homogène est 1,5-cyclooctadiène(pyridine)(tricyclohexylphosphine) iridium (I), hexafluorophosphate ou norbornadiène-1,4-bis-(diphénylphosphino)-butane-rhodium (I)-hexafluoroborate;
le solvant organique est le dichlorométhane, chloroforme ou chlorobenzène; à une température d'environ 25°C.

7. Procédé selon la revendication 6 dans lequel:
R₃ est CH₃CH₂;
R₄ est H, CH₃ ou CH₂OSi(Me)₂t-C₄H₉;
R₅ est H ou CH₂OSi(Me)₂t-C₄H₉;
à condition qu'au moins l'un de R₄ ou R₅ est H; le catalyseur est 1,5-cyclooctadiène (pyridine) (tricyclohexylphosphine) iridium (I) hexafluorophosphate;
le solvant est le dichlorométhane;
le pourcentage molaire de catalyseur au diène est 2,5 pourcent;
et le composé I(b) préparé est choisi dans le groupe dans lequel :
a. R₂ est CH₃, R₄ est CH₃, R₅ est H;
b. R₂ est H, R₄ est CH₃, R₅ est H;
c. R₂ est CH₃, R₄ est CH₂OSi(Me)₂t-C₄H₉, R₅ est H;
d. R₂ est CH₃, R₄ est H, R₅ est CH₂OSi(Me)₂t-C₄H₉;
e. R₂ est H, R₄ est CH₂OSi(Me)₂t-C₄H₉, R₅ est H;
f. R₂ est H, R₄ est H, R₅ est CH₂OSi(Me)₂t-C₄H₉.

8. Procédé selon la revendication 1 dans lequel le composé préparé a la formule I(d) : 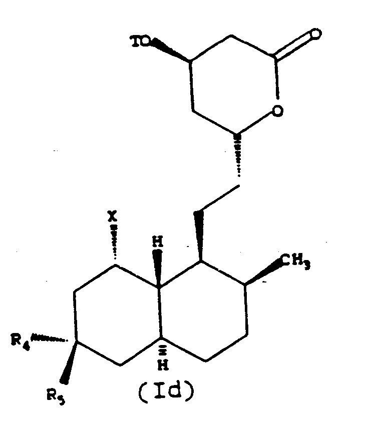 dans laquelle
T est t-C₄H₉(CH₃)₂Si;
X est OH, NH₂ ou OR₆; dans laquelle R₆ est alkyle C₁₋₅, cycloalkyle C₃₋₇, phényl(alkyle C₁₋₃), ou phényl(alkyle C₁₋₃) substitué dans lequel le substituant est un halogène, CF₃ ou CN;
R₄ est H, CH₃, CH₂OSi(Me)₂t-C₄H₉ ou OSi(Me)₂t-C₄H₉;
R₅ est H, CH₂OSi(Me)₂t-C₄H₉ ou
OSi(Me)₂t-C₄H₉;
à la condition que si l'un de R₄ et R₅ est CH₂OSi(Me)₂t-C₄H₉ l'autre est H;
le catalyseur métallique homogène est 1,5-cyclooctadiène(pyridine)(tricyclohexylphosphine)iridium(I), hexafluorophosphate ou norbornadiène-1,4-bis-(diphénylphosphino)-butane-rhodium(I)-hexafluoroborate;
le solvant organique est le dichlorométhane, chloroforme ou chlorobenzène; à une température d'environ 25°C.

9. Procédé selon la revendication 8 dans lequel:
X est OH;
le solvant est le dichlorométhane;
le catalyseur est 1,5-cyclooctadiène (tricyclohexylphosphine) (pyridine) iridium (I)-hexafluorophosphate;
le gaz hydrogène est à une pression de 2,76x10⁵Nm⁻² (40 psi);
la température est d'environ 25°C;
et le composé I(d) préparé est choisi dans le groupe dans lequel :
a. R₄ est CH₃, R₅ est H;
b. R₄ est CH₂OSi(Me)₂t-C₄H₉, R₅ est H;
c. R₄ est H, R₅ est CH₂OSi(Me)₂t-C₄H₉;
d. R₄ est OSi(Me)₂t-C₄H₉, R₅ est H;
e. R₄ est H, R₅ est OSi(Me)₂t-C₄H₉;
f. R₄ est CH₃, R₅ est OSi(Me)₂t-C₄H₉.
